# EUROPEAN PATENT SPECIFICATION

(11) **EP 2 598 132 B1**
(45) Date of publication and mention of the grant of the patent: **17.12.2014**
(21) Application number: 11738830.6
(22) Date of filing: 27.05.2011
(51) Int. Cl.: A61K 31/192, A61K 31/716, A61K 45/06, A61K 36/28, A61K 36/48, A61P 31/22, A61K 9/00, A61K 9/06, A61K 9/70, A61K 47/36

(54) **COMPOSITION FOR THE TREATMENT AND PREVENTION OF HERPES SIMPLEX LABIALIS**
ZUSAMMENSETZUNG ZUR BEHANDLUNG UND PRÄVENTION VON HERPES SIMPLEX LABIALIS
COMPOSITION POUR LE TRAITEMENT ET LA PRÉVENTION DE L'HERPÈS SIMPLEX LABIALIS

(30) Priority: 27.07.2010 IT FI20100159
(43) Date of publication of application: 05.06.2013
(73) Proprietor: Pasquali S.r.L., 50019 Sesto Fiorentino (Fl) (IT)
(72) Inventor: PASQUALI, Costantino, 50125 Firenze (IT)
(74) Representative: Mincone, Antimo
(86) International application number: PCT/IT2011/000179
(87) International publication number: WO 2012/014238

(56) References cited:
- WO-A1-2008/010241
- WO-A1-2010/045969
- WO-A2-2007/059441
- WO-A2-2008/134712
- GB-A- 2 140 809
- DATABASE WPI Week 199510 Thomson Scientific, London, GB; AN 1995-070296 XP002627027, & JP 6 345748 A (SANWA KAGAKU KENKYUSHO CO) 20 December 1994 (1994-12-20)

## Description

The present invention relates to a composition for the treatment and prevention of herpes simplex labialis.

It is known that herpes simplex is a type of virus belonging to the *herpesviridae* family and is the cause of two forms of herpes: HSV I and HSV II. The first form, remarkably widespread, that is responsible for the appearance of characteristics fever vesicles that usually affect the facial skin (lips, nose), is also called herpes simplex labialis. Less frequently, the vesicles may appear on the nasal mucosa (in particular, outside or inside the nostrils), on the cheeks or the palate. Initially, the herpes labialis provokes a slight tingling and a feeling of warmth on a reddened point of the lip. Within a few hours, some vesicles, often painful, begin to develop in the same area of the starting point. Once the inflammatory process is ended, the vesicles caused by herpes labialis dry up and form a small yellowish crust that disappears within 7-10 days even without any treatment. The appearance of herpes labialis can coincide with a state of malaise, nervousness, fever, after the onset of menstruation, stress, intense exposure to sunlight or after eating certain foods. The infection caused by herpes labialis can easily re-occur because the virus survives within cells and is not removed even with the use of effective medicines. The treatment usually involves the use of topical medicines with a basis of antivirals (aciclovir, famciclovir and valaciclovir) which however are able to reduce the infection without eliminating it completely.

The main purpose of the present invention is to propose a composition that allows to effectively treat and prevent the onset of the herpes simplex labialis.

This result is reached, in accordance with the present invention, by adopting the idea of making a composition having the characteristics specified in claim 1. Other features of the present invention are the subject of the dependent claims.

Thanks to the present invention it is possible to effectively treat and prevent the onset of herpes simplex labialis. Furthermore, a composition in accordance with the invention accelerates the healing of injuries, relieves the associated symptoms, and is easy to apply. In particular, thanks to the present invention a bio-film is formed, that prevents the vesicles of the lesion from expanding, protects the lesion from external aggressions, and avoid the pain due to dryness, air, heat, etc., and is transparent, thus providing a significant advantages in comparison to the previously mentioned active principles (acyclovir and the like) that are of a very visible white color.

These and further advantages and features of the present invention will be more and better understood by each technical of the field thanks to the following description that provides possible examples of realization of the invention, not to be meant in a restrictive sense.

A composition in accordance with the present invention is in hydrogel form and includes aqua, sorbitol, xanthan gum, panthenol, echinacea angustifolia root extract, mimosa tenuiflora bark extract, sodium carboxymethyl betaglucan, allantoin, glycyrrhetinic acid, benzyl alcohol, dehydroacetic acid, disodium EDTA.

More specifically, a composition in accordance with the present invention can be formulated as shown in the following table A.

Preferably, the amount of the mentioned substances are those indicated in table B.

The active substances act synergistically to prevent and combat labial herpes infections through four different mechanisms:
1. formation of a protective film against external aggression and against the spread of the virus to the adjacent tissues (panthenol)
2. reinforcing action of the immune system to act against external aggression (carboxymethyl-betaglucan, Echinacea angustifolia root extract)
3. action favoring the physiological scarring (as allantoin, Echinacea angustifolia root extract)
4. soothing action (allantoin, panthenol)

The composition is applied on the lips and forms a protective film. The composition can be applied several times during the day.

**TABLE A**

| **Substances** | **INCI NAME** | **Function** | **%** |
|---|---|---|---|
| Dried ext. Echinacea tit. 4% echinacoside | Echinacea angustifolia root extract | Immunostimulant, anti-inflammatory | 0,25-1% |
| Ext. glicolico mimosa | Mimosa tenuiflora bark extract | Cicatrizing, protective, soothing, antibacterial | 2-4% |
| Carbossimetil betaglucano sol. 2% | Aqua, sodium carboxymethyl betaglucan | Immunostimulant | 1-5% |
| Allantoina | Allantoin | Skin softener, soothing | 0.3-0,5% |
| Sorbitolo sol. 70% | Sorbitol, aqua | Humectant, flavor, conditioning | 5-20% |
| Gomma xantana | Xanthan gum | Gelling | 1-3% |
| Isocide BAS | Benzyl alcohol, dehydroacetic acid | Preservative | 0,5-2% |
| EDTA disodico | Disodium EDTA | Sequestering | 0,1-0,2% |
| Acido 18 β glicirretico | Glycyrrhetinic acid | Anti-inflammatory | 0,1-1% |
| Pantenolo | Panthenol | Moisturizing, film forming, soothing | 0,5-3% |
| Acqua depurata | Aqua | Solvent | to 100 |

**TABLE B**

| **Substances** | **INCI NAME** | **Function** | **%** |
|---|---|---|---|
| Dried ext. Echinacea tit. 4% echinacoside | Echinacea angustifolia root extract | Immunostimulant, anti-inflammatory | 0,25 |
| Est. glicolico mimosa | Mimosa tenuiflora bark extract | Cicatrizing, protective, soothing, antibacterial | 2,5 |
| Carbossimetil betaglucano sol. 2% | Aqua, sodium carboxymethyl betaglucan | Immunostimulant | 2 |
| Allantoina | Allantoin | Skin softener, soothing | 0,3 |
| Sorbitolo sol. 70% | Sorbitol, aqua | Humectant, flavor, conditioning | 10 |
| Gomma xantana | Xanthan gum | Gelling | 2,2 |
| Isocide BAS | Benzyl alcohol, dehydroacetic acid | Preservative | 1 |
| EDTA disodico | Disodium EDTA | Sequestering | 0,1 |
| Acido 18 β glicirretico | Glycyrrhetinic acid | Anti-inflammatory | 0,15 |
| Pantenolo | Panthenol | Moisturizing, film forming, soothing | 1 |
| Acqua depurata | Aqua | Solvent | to 100 |

## Claims

1. Composition for use in the treatment and prevention of herpes simplex labialis **characterized by** the fact that it comprises, in combination, the following substances: Mimosa tenuiflora bark extract, Aqua, sodium carboxymethyl betaglucan, Echinacea angustifolia root extract and Glycyrrhetinic acid.

2. Composition for use according to claim 1 **characterized in that** it comprises, in combination, aqua, sorbitol, xanthan gum, panthenol, Echinacea angustifolia root extract, Mimosa tenuiflora bark extract,sodium carboxymethyl betaglucan, allantoin, glycyrrhetinic acid, benzyl alcohol, dehydroacetic acid, disodium EDTA.

3. Composition for use according to claim 1 **characterized in that** it comprises the following amounts of the said substances: Mimosa tenuiflora bark extract 2-4%, Aqua, sodium carboxymethyl betaglucan 1-5%, Echinacea angustifolia root extract 0.25-1%and Glycyrrhetinic acid 0.1-1%.

4. Composition for use according to claim 1 **characterized in that** it comprises the following amounts of the said substances: Mimosa tenuiflora bark extract 2.5%, Aqua, sodium carboxymethyl betaglucan 2%, Echinacea angustifolia root extract 0.25%and Glycyrrhetinic acid 0.15%.

5. Composition for use according to claim 1 **characterized in that** it is in the hydrogel form.

6. Composition for use according to claim 1 **characterized in that** it is transparent.

## Patentansprüche

1. Zusammensetzung zur Verwendung bei der Behandlung und Vorbeugung von Herpes simplex labialis, **dadurch gekennzeichnet, dass** sie in Kombination die folgenden Substanzen umfasst: Rindenextrakt von Mimosa tenuiflora, Aqua, Natriumcarboxymethyl-Betaglucan, Wurzelextrakt von Echinacea angustifolia und Glycyrrhizinsäure.

2. Zusammensetzung zur Verwendung nach Anspruch 1, **dadurch gekennzeichnet dass** sie in Kombination folgende Bestandteile umfasst: Wasser, Sorbitol, Xanthanlösung, Pantothenol, Wurzelextrakt von Echinacea angustifolia, Rindenextrakt von Mimosa tenuiflora, Natriumcarboxymethyl-Betaglucan, Allantoin, Glycyrrhizinsäure, Bencylalkohol, Dehydroessigsäure, Dinatrium-EDTA.

3. Zusammensetzung zur Verwendung nach Anspruch 1, **dadurch gekennzeichnet, dass** sie folgende Substanzmengen umfasst: Rindenextrakt von Mimosa tenuiflora 2 % - 4 %, Aqua, Natriumcarboxymethyl-Betaglucan 1 % - 5 %, Wurzelextrakt von Echinacea angustifolia 0,25 % - 1 % und Glycyrrhizinsäure 0,1 % - 1 %.

4. Zusammensetzung zur Verwendung nach Anspruch 1, **dadurch gekennzeichnet, dass** sie die folgenden Substanzmengen umfasst: Rindenextrakt von Mimosa tenuiflora 2,5 %, Aqua, Natriumcarboxymethyl-Betaglucan 2 %, Wurzelextrakt von Echinacea angustifolia 0,25 % und Glycyrrhizinsäure 0,15 %.

5. Zusammensetzung zur Verwendung nach Anspruch 1, **dadurch gekennzeichnet, dass** sie in Form eines Hydrogels vorliegt.

6. Zusammensetzung zur Verwendung nach Anspruch 1, **dadurch gekennzeichnet, dass** sie transparent ist.

## Revendications

1. Composition pour l'usage dans le traitement et la prévention de l'herpès simplex labial, **caractérisée par le fait que** elle comprend, en combinaison, les substances suivantes: extrait de l'écorce de Mimosa tenuiflora, Aqua, sodium carboxyméthy bêta-glucane, extrait de racine de Echinacea angustifolia et de l'acide glycyrrhétinique.

2. Composition pour l'usage selon la revendication 1, **caractérisée en ce que** elle comprend, en combinaison, aqua, sorbitol, gomme xanthane, panthénol, extrait de racine de Echinacea angustifolia, extrait d'écorce de Mimosa tenuiflora, sodium carboxyméthy bêta-glucane, allantoïne, acide glycyrrhétinique, alcool benzylique, acide déhydroacétique, EDTA disodique .

3. Composition pour l'usage selon la revendication 1, **caractérisé en ce que** elle comprend les quantités suivantes des dites substances: extrait d'écorce de Mimosa tenuiflora 2-4%, Aqua, sodium carboxyméthy bêta-glucane 1-5%, extrait de racine d'Echinacea angustifolia 0,25-1% et acide glycyrrhétinique 0,1-1%.

4. Composition selon la revendication 1, **caractérisé en ce qu'**il comprend les quantités suivantes de substances dites: Mimosa tenuiflora extrait d'écorce de 2,5%, Aqua, la carboxyméthylcellulose de sodium 2% bêta-glucane, Echinacea angustifolia extrait de racine de 0,25% et 0,15% d'acide glycyrrhétinique.

5. Composition pour l'usage selon la revendication 1, **caractérisé en ce que** elle se présente sous forme d'hydrogel.

6. Composition pour l'usage selon la revendication 1, **caractérisé en ce que** elle est transparent.
